# EUROPÄISCHE PATENTANMELDUNG

(11) **EP 1 818 315 A1**
(43) Veröffentlichungstag der Anmeldung: **15.08.2007**
(21) Anmeldenummer: 06002488.2
(22) Anmeldetag: 07.02.2006
(51) Int. Cl.: C02F 11/04

(54) **Dynamischer Mikromischer**

(71) Anmelder: Von Nordenskjöld, Reinhart, Dr.-Ing., 85658 Egmating-Münster (DE)
(72) Erfinder: Von Nordenskjöld, Reinhart, Dr.-Ing., 85658 Egmating-Münster (DE)
(74) Vertreter: Zipse Habersack Kritzenberger

(57) **Zusammenfassung**

Die Erfindung betrifft ein Verfahren zur feinvolumigen Vermischung von Substraten in Reaktoren mit Flachböden durch konstante oder wechselnde flächenartige bodennahe Gaseinblasung, wobei die Gaseinblasung in, den Boden im Wesentlichen vollständig abdeckende Sektionen aufgeteilt ist, die steuerbar im dynamischen Wechsel mit Gas ganz oder teilweise beaufschlagbar sind, derart, dass an jedem Volumenpunkt des Reaktors eine lokale Mikroturbulenz entsteht.

## Beschreibung

### GEBIET DER ERFINDUNG

Beiliegende Patentanmeldung befasst sich mit der Durchmischung von Großbehälter-Inhalten zur Optimierung biochemischer Abläufe bei anaeroben Prozessen, insbesondere bei anaeroben Prozessen zur Generierung von Biogas. Solche Biogas-Generierungsprozesse werden mehr und mehr installiert und betrieben, ermöglichen sie doch organische Stoffe unter Energiegewinnung biologisch abzubauen, d.h. durch Bildung von Biogas (im Wesentlichen Methan). Die Anwendung reicht u.a. von der Reinigung von Abwässern (Beispiel Skjöldgas-System des Anmelders gemäß EP 0 998 430) über den Abbau von Abwasserinhaltsstoffen in Kombination mit organischen Rückständen (Beispiel Skjöldgas - FFF-System des Anmelders (Fest-Flüssig-Fermentation gemäß EP 1 419 995)) bis hin zur anaeroben Vergärung von speziell dafür angebauten Pflanzen, wie z.B. Mais, Roggen, Gras u.Ä., die oftmals siliert ganzjährig als sog. NOWARO (nachwachsende Rohstoffe) dem Biogasreaktor zugeführt werden. Dem sind häufig unter Verwendung des produzierten Gases eine Verstromung mit Wärmenutzung (BHKW) zugeordnet.

### Der Prozess

Bekannterweise verläuft der Prozess zur Biogasgewinnung grob beschrieben über die Bildung von organischen Säuren zur Bildung von Methan+ (+ bedeutet sonstige Gaskomponenten wie CO₂, H₂S, Dampf etc.). Das Besondere an diesem Prozess ist u.a., dass beide Schritte, d.h. die Bildung organischer Säuren einerseits und die Methanisierung andererseits, möglichst getrennt voneinander ablaufen sollten, weil die mit der Versäuerung (man nennt diese auch Vorversäuerung) verbundenen, niedrigen pH-Werte von ca. 4-6 für die anschließend aktiven Methanbakterien - die bevorzugt bei pH-Werten um 7 leben und ihre volle Aktivität entwickeln - bei einem pH-Wert ≥ 6,7 weitestgehend geschwächt bzw. getötet werden und damit die biochemische Umwandlung der (organischen) Säuren in Methan+ zum Erliegen kommt.

Um den Prozess zu optimieren, d.h. aus einem kg organischer Substanz möglichst viel Gas (ca. 0,6 m³ Gas/kg OTS) mit hoher Qualität (z.B. Methangehalt von 65-80%) zu erhalten, sind viele Aspekte zu beachten, die auch noch nicht vollständig erforscht sind.

### STAND DER TECHNIK

Zum Stand der Technik im beschriebenen Aufgabengebiet werden nachfolgend die am meisten zum Einsatz kommenden Methoden, insbesondere die der Bewegung des Reaktorinhalts beschrieben, denn ihre optimale Gestaltung hat wesentlichen Anteil an der erfolgreichen Prozeßdurchführung.
a) Anaerobe Anlagen mit Schlammrückführung werden in der Regel durch Zupumpen von Rohwasser einschließlich dem Schlamm aus der Nachklärung in Bewegung gehalten, d.h. es wird gleichzeitig die Materialzufuhr eingemischt, insbesondere wenn es sich um Reaktoren mit aktiven Schlammschichten aus sog. "Pellets" handelt. Die direkte Zugabe kann dabei durch den Außenmantel oder vom bzw. am Boden der Reaktoren aus erfolgen.
b) Reaktoren von kommunalen Kläranlagen sammeln die schwereren Bestandteile durch ihre trichterförmigen Böden der zumeist eiförmigen Reaktoren, um sie von dort durch die zentrale Einpressung von Gas (Einblasringrohre) wieder aufzuwirbeln, wobei eine Ringströmung (ähnlich der Form eines Magnetfeldes) angestrebt wird. Auch kommen einzelne Einblaslanzen zum Einsatz.
c) Landwirtschaftliche Anlagen verwenden in der Regel für:
   1. kleinere Anlagen bis ca. 500 m³ Reaktorvolumen schnelle (ca. 100 U/Min. und mehr) Propellerrührwerke;
   2. größere Anlagen von bis zu 3.500 m³ Reaktorvolumen hängende, langsam laufende (ca. 13-20 U/Min.) Großrührwerke mit einigen Metern Durchmesser.
   Beide Typen sind durch hohe Propeller bzw. Flügelkantengeschwindigkeiten von ca. 1,5-3 m/s gekennzeichnet und führen darüber hinaus zu mehr oder weniger großen Totzonen und Ablagerungen.
d) Seltener werden insbesondere bei Reaktoren mit größeren Durchmessern um 15 m und flachen Böden Senkrechtlanzen mit unten in Bodennähe liegenden großen Ausblasöffnungen zur Gaseinbringung eingesetzt.

Weil der Prozess an sich der Menschheit beispielsweise in China seit langem bekannt ist, aber erst heute in großem Umfang zur Anwendung gelangt, sind in der Praxis - wie oben gezeigt - verschiedenste technische und verfahrenstechnische Lösungen in Gebrauch, bzw. werden in großer Stückzahl mit mehr oder weniger großem Erfolg realisiert, ohne voll entwickelt zu sein. Auch schon gut bekannte Zusammenhänge, wie etwa die mechanische Empfindlichkeit der Bakterien, werden nicht genügend beachtet, was dann zu schlechter bzw. geringer Wirtschaftlichkeit oder Verlusten führt. So werden vor allem die nachfolgenden Zusammenhänge, mit denen sich die vorliegende Erfindung befasst, oft nicht beachtet:
a) Das gebildete Gas muss nach seiner Bildung so schnell wie möglich aus dem Substrat von Einzelbakterien weg entfernt werden, u.a., weil im Gas auch für die Methanbakterien giftige Stoffe (z.B. H₂S) enthalten sind; die Bakterien dürfen nicht im Gas "ersticken" (Sättigung).
b) Bakterielle Reaktions-Vorgänge verlaufen immer über viele Schritte, die selten genau erforscht werden können und die in der Regel durch Gruppierungen von Bakterien und ihr Zusammenwirken optimal vonstatten gehen. Eine intensive (mechanische bzw. hydraulisch-mechanische) Behandlung erschwert bzw. verhindert diese Reaktionen, die Voraussetzung für den Gesamtprozess sind.
c) Insbesondere der Biogasgenerierungsprozess erfordert, dass das Substrat nicht einer ständigen Umwälzung oder eines ständigen Umrührens unterliegt, sondern nur so weit erforderlich und schonend durchmischt wird. Es muss das Ziel der "Bewegung" im Reaktor sein, Säuren und Methanbakterien zusammenzuführen, Gas aus dem Gemisch zu entfernen, Ablagerungen zu verhindern und gleichmäßige Temperaturzustände zu erhalten, und das Ganze in homogener und schonender Weise! (Schafe werden kaum fressen, wenn man sie ständig über die Weide jagt).
d) Die Methanisierung verläuft nach klassischer Auffassung am optimalsten in zwei Temperaturbereichen, und zwar bei ca. 34° C (mesophiler Bereich) und bei ca. 53° C (thermophiler Bereich). Insbesondere der zweite Bereich ist in einer engen Temperaturbreite angesiedelt und schon geringe Temperaturschwankungen von zwei bis drei Grad Celsius können die Effizienz des Prozesses um bis ca. 40% mindern. Da auch die Vermehrung der Bakterien - die bei den Prozessen ohne Schlammrückführung essentiell und ein ständiger Verlust durch den Austrag zu berücksichtigen ist - in ähnlich drastischer Weise von Temperaturschwankungen abhängt, ist in allen Bereichen des Prozesses eine schonende und auf höchste Vergleichmäßigung ausgerichtete Temperaturführung erforderlich. Auch hier sind Produktführung und Dynamik im Reaktor von entscheidendem Einfluss, da nur bei ihrer optimalen Gestaltung die eben beschriebenen Negativeffekte schwankender und ungleichmäßiger Temperaturverteilungen vermieden werden.
e) Eine Biogasanlage hat einen Eigenenergiebedarf in Form von Wärmeenergie zur Aufrechterhaltung der Reaktortemperatur und in Form von elektrischer Energie für die verschiedensten Antriebe insbesondere der Umwälzaggregate; diese gilt es zu minimieren.
   Wenn man den ganzen Reaktor umwälzt, erhöht sich der Wärmeübergang durch die auftretenden Wandströmungen mit entsprechendem Anstieg der Wärmeverluste. Weiterhin geht die übliche Umwälzung, die indirekt der Vermischung dienen soll, energetisch durch "ihre Fernwirkung" über den Bedarf letzterer deutlich hinaus, und dieses "Mehr" kann vermieden werden, wenn man sich nur auf die nötige Vermischung konzentriert.
f) Rührwerke zur Umwälzung des Reaktors sind ein bewegliches Teil im Reaktor und stellen ein hohes Risikopotential (Dauerbetrieb!) dar. Bei Defekten wie z.B. auf Grund der verschiedensten Schwingungen (Füllungsgrad etc.) auftretenden Wellenbrüche muss der Reaktor stillgelegt, das Rührwerk ausgebaut, repariert etc. werden, also insgesamt eine unbefriedigende viel Geld kostende Lösung.
g) Biogasanlagen durchlaufen verschiedenste Betriebszustände, so dass ihre Mischeinrichtungen anpassungsfähig sein müssen; die derzeitige Umwälzeinrichtungen sind nur begrenzt regelbar. Dies ist ein großer Nachteil, denn verschiedene Produkte, verschiedene Füllungsgrade des Reaktors und verschiedene Betriebszustände - z.B. Anfahren, Nachheizen u.Ä. - bedürfen der vollen Anpassung der Umwälzung bzw. des Mischens; nur so kann man sich zur Prozessoptimierung jeweils anpassen.
h) "Blockströmungen", d.h. ganze, in sich gar nicht oder nur wenig durchmischte "Flüssigkeitsblöcke", müssen vermieden werden; solche treten u.a. auf, wenn ein Zentralrührwerk den Inhalt eines stehenden Rundreaktors "im Kreis bewegt", wie es im Normalbetrieb oder z.B. etwa bei höheren Viskositäten oft der Fall ist.
i) Schließlich sind alle Prozessschritte homogen, vollständig und eindeutig zu gestalten. Nur so sind einwandfreie und repräsentative Messwerte als Basis für eine erfolgreiche und sichere Steuerung zu gewinnen und nur so läuft der Prozess optimal.
j) Es sind unbedingt Kurzschlussströmungen vom Zu- zum Ablauf zu vermeiden. Diese treten besonders auf, wenn man wie üblich rührt und weniger mischt und dadurch Strömungsfäden bildet. Ungenügender Abbau und ungenügende Effizienz sind die Folge.
k) Bei der derzeitigen Bauweise von Reaktoren wird z.T. unnötig teuer gebaut, z.B. wenn ein Behälterdach und -mantel große Kraft-Momente und das Gewicht eines Zentralrührwerks zusätzlich aufnehmen müssen.
l) Gerade bei umwälzend arbeitenden Einrichtungen treten immer wieder Schwimmschichten auf, die den Gesamtprozess empfindlich stören und zum Beispiel auch das Entfernen der Gase aus dem Volumen verhindern oder behindern.
m) Vertikal nach unten gerichtete Strömungen im Geschwindigkeitsbereich aufsteigender Gasblasen (0,05-0,3 m/s) sind zu vermeiden, da sie mit aufsteigenden Gasblasen einen Stau bilden und zwar mit prozesshemmender Wirkung.
n) Methanbakterien sind temperaturempfindlich, so dass sie bei zu hohen Temperaturen - ca. > 50° C - von Heizeinrichtungen (z.B. Heizrohren) und auch eingeblasenem Gas geschädigt oder getötet werden - die negativen Folgen sind klar

### KERNGEDANKE DER ERFINDUNG

Die erfindungsgemäß vorgeschlagene Verfahrensweise lehnt die übliche Reaktorumwälzung ab und setzt demgegenüber an einer dynamischen, d.h. wechselnden, flächigen Reaktormischung an. Kerngedanke ist damit die Abkehr von einer durch die Zwischenschaltung des Umwälzprozess mittelbar erhaltene Substrat-Mischung und demgegenüber eine Hinwendung und Realisierung einer an jedem Volumenpunkt des Reaktors erzielbare Mikroturbulenz im Substrat, sodass dies in lokalen Mikrobereichen durchmischt wird. Dies wird erreicht durch feinblasiges Einbringen von Gas über im Wesentlichen die gesamte horizontale Querschnittsfläche des Reaktors, beispielsweise über den gesamten Reaktorboden. Das eingebrachte Gas erfüllt damit ausschließlich physikalisch- mechanische Zwecke einer Durchmischung und ggf. Aufsammlung von Gasblasen, die durch die Biogas-Generierung entstehen. Chemische Reaktionen werden vordergründig durch das erfindungsgemäß eingebrachte Gas nicht angestrebt, sind aber nicht auszuschließen und erste Beobachtungen lassen eine positive Wirkung vermuten.

Entsprechend dem Erfindungsgedanken werden z.B. bei Zylinderreaktoren mit ebenen Böden diese aufgeteilt in einzelne Bodenbereiche - 1/8 bis 1/4 der Bodenfläche - und mittel-, bzw. feinblasig mit Gas bodennah angeströmt, um durch den so erzeugten Blasenschwarm eine milde aber intensive und vollständige Vermischung des Reaktorvolumens quasi im Mikrobereich in jedem Volumenpunkt zu bewirken, wobei sich die Einzelzonen z.B. im umlaufenden Kreisrhythmus abwechseln können, um somit das gesamte Reaktorvolumen im Wechsel nacheinander vollständig zu erreichen. Das verwendete Gas ist vorteilhafterweise das im Reaktor generierte Biogas, es kann aber auch CO₂ oder Stickstoff oder jedes inerte Gas verwendet werden.

Der Wechsel unterschiedlich angesteuerter Sektionen kann sowohl durch relative langsame Fortbewegung - z.B. kreisend - der Eintragseinrichtung als auch durch rhythmisches Umschalten von Segmentbereichen einer im Wesentlichen den gesamten Bodenbereich umfassenden Einblaseinrichtung erzeugt werden. Diese neue Vorgehensweise hat eine verfahrenstechnische Breitbandwirkung für alle nötigen Verfahrenseffekte, denn sie
1. treibt vorhandene Gasblasen durch Anlagerung an das eingeblasene Gas vollständig nach oben aus dem Reaktorvolumen hinaus,
2. arbeitet äußerst schonend und stört die biologischen Vorgänge nicht,
3. führt im gesamten Volumen Nährstoffe und Bakterien mittels schonender Durchmischung zusammen,
4. erreicht jeden Reaktorteil und vergleichmäßigt die Temperaturen eindeutig, und eine einwandfreie Messung und Regelung wird durch sie ermöglicht,
5. minimiert durch geringe Wandströmung die Wärmeverluste und benötigt durch nacheinander folgende Sektionsbehandlung nur geringe Energiemengen,
6. kann andererseits Sektionen auch bei ungewöhnlichen Betriebszuständen sozusagen durch "Konzentration" der Gasmengen so stark beaufschlagen, dass auch eine Umwälzung praktiziert werden kann,
7. ermöglicht auch, wenn erforderlich, Umwälzung und Feinmischung in Folge einzusetzen,
8. kann auch ohne jegliche bewegliche Teile im Reaktor verwirklicht werden,
9. ist bezüglich spezifischer Flächenbelastung, Folge und Art der dynamisch wechselnden Zonen voll regelbar,
10. lässt Blockströmungen, d.h. Strömungen von ganzen Volumenblöcken nicht aufkommen,
11. vermeidet Kurzschlussströmungen vom Zulauf zum Ablauf, denn die "Mischungsfelder" wirken wie Barrieren; sie wandeln das gesamte Strömungsfeld quasi in optimaler Weise in eine Art Pfropfenströmung um,
12. belastet durch ihre mittel-, bzw. feinblasige Gasmischungsfelder die Reaktoren mechanisch nur geringfügig - z.B. treten Rührwerksmomente und Gewichte gar nicht erst auf,
13. bewältigt kritische Situationen bei Teilfüllung bzw. Viskositätsänderungen - die bei Rührwerken oftmals zu Schwingungen und Wellenbrüchen führen, problemlos,
14. erlaubt durch Verwendung des Gases als eigentlichen Energieträger bei den üblicherweise zusammenstehenden Einheiten, z.B. alle am Standort zur Verfügung stehenden Druckgasmengen vorübergehend in einem Reaktor, der z.B. durch zu viel Roggen zu viskos wurde, zu konzentrieren,
15. zerstört Schwimmschichten, d.h. geschlossene auftreibende Feststoff-Verbünde, bzw. lässt sie gar nicht erst aufkommen. Schwimmschichten können dabei durch gezielte Ansteuerung der relevanten Sektion, die die Schwimmschicht enthält, zerstört werden, indem soviel Gas in dieser Sektion eingeblasen wird, dass durch das aufsteigende Gas die Schwimmschicht platzt;
16. durch die erfindungsgemäße Lösung ist die Betriebsfunktion auch bei Teilfüllung gewährleistet, indem die eingetragene Gasmenge auf die Substratbedingungen angepasst wird.
17. kann durch stufenlose Anpassung der eingebrachten Gasmenge den unterschiedlichsten Reaktorbedingungen gerecht werden.
18. minimiert die eingesetzte Energie durch Kühlung der durch die Verdichtung teilweise stark erhitzten Gase, indem diese Kühlung am Stoffstrom oder in indirekter Weise im Rahmen der Beheizung erfolgt, so dass die abgegebene Energie dem eigentlichen Prozess (Wärmeverluste!) zu Gute kommt.

Beim Einsatz der erfindungsgemäßen Einrichtung zur Durchmischung erhöhte sich in einem Fall die Methanproduktion um über 35%. Ferner gelang es sogar mit eben der erfindungsgemäßen Einrichtung einen erfolgreichen Prozess schon bei ca. 25° C über ca. 10 Monate stabil zu betreiben und dies bei hoher Ausbeute von bis zu 0,6 m³ Gas/Δkg OTS, sowie bei hohem Methangehalt um ca. 75%.

Schließlich ist es möglich, mehrere Reaktoren mit einzelnen Gasversorgungs-Quellen in einem Verbund zusammenzuschalten. Das ermöglicht es, bei konstanter Blasleistung des/der Verdichter eine erhöhte Gaszufuhr in einem Reaktor durch eine Gasmengen-Reduzierung an einem anderen Reaktor hervorzurufen.

Zur Unterstützung des oben erläuterten Verfahrens ist es zweckdienlich, dass der Substrateintrag in den Reaktor im oberen Bereich im Wesentlichen gleichmäßig über die gesamte horizontale Reaktor-Querschnittsfläche erfolgt, um eine vertikal nach unten gerichtete Pfropfenströmung des Substrats zu erhalten. Damit ist sichergestellt, dass die Verweilzeit für jedes Substratteilchen annähernd dieselbe und optimal lange ist.

Die Erfindung wird im Folgenden unter Bezugnahme auf die beigefügten Zeichnungen näher erläutert. In diesen zeigen,
- Fig. 1: eine Gegenüberstellung eines herkömmlichen Biogasreaktors, wie er im Stand der Technik gemäß obiger Beschreibung verwendet wird (linke Seite der Figur), mit einem Biogasreaktor, bei dem das erfindungsgemäße Verfahren angewendet wird (rechte Seite der Zeichnungsfigur);
- Fig. 2: den Biogasreaktor mit Mitteln zur Durchführung des erfindungsgemäßen Verfahrens im Detail.

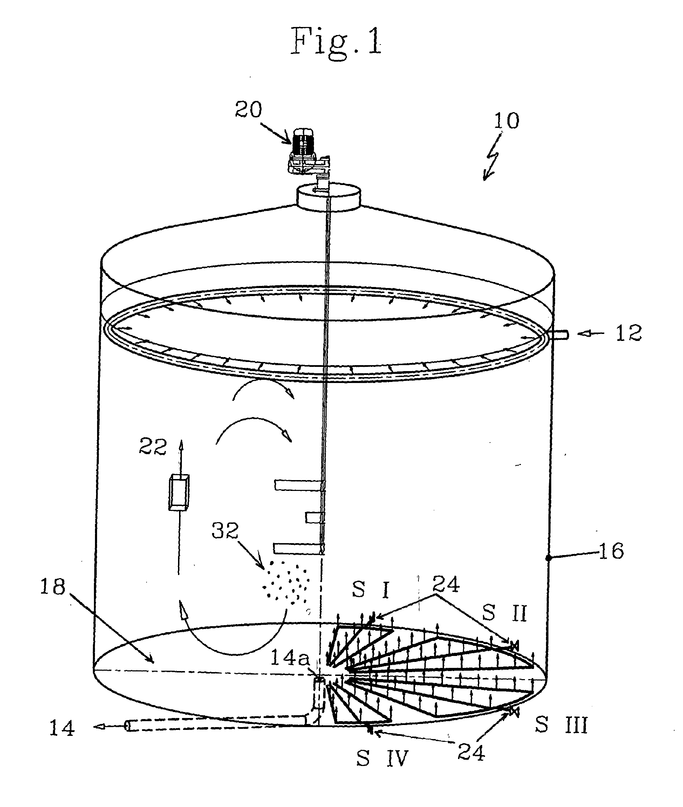

In Figur 1 ist ein kreisrunder Biogasreaktor 10 in perspektivischer Ansicht gezeigt, bei dem im linken Bereich der Zeichnungsfigur ein herkömmliches Umwälzverfahren (in der Fig. nur teilweise dargestellt) mittels einem im Stand der Technik bekannten Rührwerk 20 durchgeführt wird. Das im oberen Bereich des Reaktors im Substrateinlauf 12 einströmende Substrat wird durch das Rührwerk 20 gemäß den in der linken Zeichnungshälfte aufgezeigten Pfeilen umgewälzt. Nach einer im Durchschnitt ausreichenden Verweilzeit im Reaktor wird das biologisch umgewandelte Substrat am Substratauslauf 14 des Reaktorbodens 18 abgelassen. Wie das in der linken Zeichnungshälfte zum Stand der Technik durch die Blockströmung 22 veranschaulicht ist, werden durch eine Umwälzung mittels Rührwerk sog. Stromfäden (siehe langer vertikaler Pfeil) gebildet, bei denen ganze Volumenblöcke von Substrat ohne innerer Durchmischung im Reaktor bewegt werden. Allerdings wird auch zulaufendes Substrat zumindest teilweise schnell zum Ablaufpunkt 14a transportiert und somit nur teilweise abgebaut. Auch kann ein Gasblasenstau 32 durch die nach unten gerichtete Strömungsrichtung im Mittelbereich auftreten

Diesem Verfahren ist auf der rechten Hälfte der Zeichnungsfigur 1 das erfindungsgemäße Sektions-Mischverfahren gegenübergestellt. Hierzu sind flächig über im Wesentlichen die gesamte horizontale Querschnittsfläche des Reaktorbodens 18 Einblaseinrichtungen für einen Gaseintrag vorgesehen. Die Verteilung von Einblasdüsen, bzw. Einblasöffnungen hat so zu erfolgen, dass durch den Gaseintrag an jedem Volumenpunkt des Reaktors eine lokale Mikroturbolenz durch die nach oben strömenden Gasblasen erhältlich ist. Das eingebrachte Gas erfüllt damit die erforderlichen physikalisch-mechanischen Zwecke einer Durchmischung und Aufsammlung von Gasblasen, die durch die Biogas-Generierung entstehen. Chemische Reaktionen werden durch das erfindungsgemäß eingebrachte Gas nicht angestrebt, können aber ggf. sogar mit positivem Effekt stattfinden. Die Einblaseinrichtungen sind zur Beaufschlagung mit Gas in einzelne Sektionen unterteilt, von denen in der rechten Zeichnungsfigur vier Sektionen S I bis S IV gezeigt sind. Der Gaseintrag erfolgt für jede Sektion über ein steuerbares Ventil 24, so dass die für jede Sektion erforderliche Gasmenge von 0 bis max. frei regelbar ist.

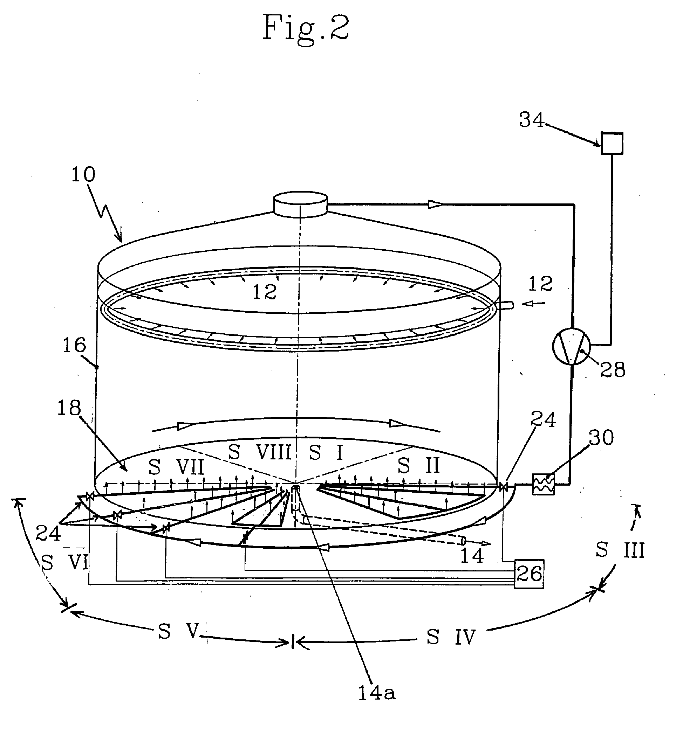

Figur 2 veranschaulicht das erfindungsgemäße Verfahren in größerem Detail. Der Reaktorboden 18 des kreisrunden Biogasreaktors 10 ist in insgesamt 8 Sektionen S I bis S VIII aufgeteilt. In jeder Sektion befinden sich so viele Einblasdüsen, wie es zum Erhalt einer lokalen Mikroturbolenz an jedem Volumenpunkt des Reaktors erforderlich ist. Das über die am Reaktorboden verteilten Einblasdüsen eingebrachte Gas verursacht eine Durchmischung des Substrats durch die nach oben steigenden Gasblasen in jedem Mikro-Volumenelement des Reaktors. Durch diese milde Mikromischung ist eine Blockströmung verhindert, d.h. es ist vermieden, dass ein Volumenblock in dem Reaktor ohne eigene Durchmischung lediglich umgewälzt wird. Damit sind auch ganze Stromfäden verhindert, d.h. zirkulierende Trassen aus Volumenblöcken.

Durch das Einblasen feiner Gasblasen am Boden des Reaktors erfolgt ein rascher Austrag der Gase, wie beispielsweise auch das schädliche H₂S, da sich die Gase an den aufsteigenden eingebrachten Gasblasen anlagern und mit diesen nach oben an die Substratoberfläche aufsteigen.

Die einzubringende Gasmenge kann durch Variationen in der Sektionsbeaufschlagung oder direkten Gasmengenregulierung 34 an die einzelnen Substrateigenschaften und diversen Betriebszustände angepasst werden. Wenn sich beispielsweise eine aus festem Anteil gebildete Schwimmschicht an der Substratoberfläche ansammelt, kann durch eine erhöhte Gasbeaufschlagung an derjenigen Sektion, oberhalb derer sich die Schwimmschicht befindet, diese zerstört werden.

Durch die insgesamt erreichten minimierten Strömungsverluste im Substrat ist der Energieeintrag geringer. Auch sind die Wärmeverluste aufgrund der geringen Wandströmung minimiert.

Wie das ferner in Figur 2 gezeigt ist, erfolgt der Gaseintrag am Boden 18 des Reaktors z.B. mittels Abzug über das am Dach des Reaktors erhältliche Biogas. Eine Gaseinblaseinrichtung 28 verdichtet das abgesogene Biogas und bläst es über Ventile 24, den einzelnen Sektionen zugeteilt, in das Substrat. Die aufgewendete Verdichtungsenergie wandelt sich zum großen Teil in Gaswärme um und heizt den Reaktor, d.h. sie wirkt doppelt. Da die Gaswärme mit zu hoher und ggf. schädlichen Temperaturhöhe verbunden sein kann, wird dies durch einen Kühler 30 vermieden, wobei die so gewonnene Wärme vorteilhafterweise ggf. an anderer unempfindlicher Stelle - z.B. in die Vorversäuerung - dem Prozess zugeführt werden kann.

Der Zustrom an Gas für die einzelnen Sektionen ist beispielhaft in Sektion S III, S V und S VI unterschiedlich gezeigt. In Sektion S III erfolgt der Gaseintrag über eine zum Mittelpunkt des Reaktors am Boden 18 geführte Leitung mit Ventil 24, durch die der Gaseintrag vom Mittelpunkt des Reaktors nach außen verteilt zur Reaktorwand 16 erfolgt. In der Sektion S V erfolgt der Eintrag über eine Leitung mit Ventil von der Zylinderwand 16 des Reaktors her; das Rohr teilt sich in drei Einblasleitungen auf. Gemäß Sektion S VI ist jede der drei gezeigten Einblasleitungen mit einem Ventil 24 versehen, wobei jede Leitung von der Zylinderwand 16 des Reaktors bis zum Mittelpunkt geführt ist. Die Ventile 24 werden mittels einer Ventilsteuerung 26 angesteuert, so dass der Gaseintrag sektionsweise und/oder in der Gasmenge regelbar ist. Die gezeigten Beispiele zeigen beispielhaft, wie flexibel das System den Reaktorformen und verschiedenen Prozessen angepasst werden kann.

Gemäß einer vorteilhaften Ausführungsform erfolgt der Substrateintrag im oberen Bereich des Reaktors 10 über einen Substrateinlauf 12 ringförmig, so dass das Substrat von der Zylinderwand des Reaktors her zu dessen Mittelpunkt gleichmäßig verteilt über die horizontale Querschnittsfläche des Reaktors einströmt. Damit wird eine vertikal nach unten gerichtete Pfropfenströmung des Substrats möglich.

Eine Gasbeaufschlagung der in die acht Zonen aufgeteilte Bodenbegasungseinrichtung kann im Wechsel erfolgen, derart, dass eine kreisförmig bewegte Gasbeaufschlagung realisiert ist, wie das durch den Langpfeil in Figur 2 gezeigt ist. Mit dem erfindungsgemäßen Verfahren wird eine vertikale Durchströmung des Substrats mittels Gasblasen hervorgerufen, die zu einer Durchmischung des Substrats in jedem Mikro-Volumensegment führt.

Der Übersichtlichkeit wegen sind in Fig. 2 die Sektion S IV nicht vollständig, sowie die Sektionen S I, S II, S VII und S VIII nicht mit Einblasrohren gezeigt.

### Bezugszeichenliste:

- 10: runder Biogasreaktor
- 12: Substrateinlauf
- 14: Substratauslauf
- 14a: Eintritt in Ablauf
- 16: Zylinderwand des Reaktors
- 18: Reaktorboden
- S: Sektion des Reaktorbodens
- 20: Rührwerk im St.d.T
- 22: Blockströmung
- 24: Ventil
- 26: Ventilsteuerung
- 28: Gasverdichter
- 30: Kühlung
- 32: Gasblasenstau
- 34: Regler (z.B. Frequenzumregler)

## Patentansprüche

1. Verfahren zur feinvolumigen Vermischung von Substraten in Reaktoren mit Flachböden durch flächenartige bodennahe Gaseinblasung,
**dadurch gekennzeichnet, dass** die Gaseinblasung in, den Boden im Wesentlichen vollständig abdeckende Sektionen (S) aufgeteilt ist, die steuerbar im dynamischen Wechsel mit Gas beaufschlagbar sind, derart, dass an jedem Volumenpunkt des Reaktors eine lokale Mikroturbulenz hervorgerufen werden kann.

2. Verfahren nach Anspruch 1,
**dadurch gekennzeichnet, dass** die Ansteuerung der Sektionen (S) zur Gasbeaufschlagung durch eine Bestimmung der Gasmenge durch die einzelnen Sektionen erfolgt.

3. Verfahren nach Anspruch 1 oder 2,
**dadurch gekennzeichnet, dass** die Ansteuerung der Sektionen (S) zur Gasbeaufschlagung im zeitlichen Wechsel erfolgt.

4. Verfahren nach Anspruch 3,
**dadurch gekennzeichnet, dass** der zeitliche Ansteuerungswechsel durch einen/mehrere frequenzgeregelte(n) Gasverdichter erfolgt.

5. Verfahren nach einem der vorangehenden Ansprüche,
**dadurch gekennzeichnet, dass** der Substrateintrag in den Reaktor im Wesentlichen gleichmäßig oben über die gesamte horizontale Reaktor-Querschnittsfläche erfolgt, um eine vertikal nach unten gerichtete Pfropfenströmung des Substrats zu ermöglichen.

6. Verfahren nach einem der vorangehenden Ansprüche,
**dadurch gekennzeichnet, dass** die in das Substrat einzubringende Gasmenge an die Substrateigenschaften angepasst ist, beispielsweise derart, dass die Gasmenge bei zunehmender Viskosität des Substrats erhöht ist.

7. Verfahren nach einem der vorangehenden Ansprüche,
**dadurch gekennzeichnet, dass** mehrere Reaktoren in einem Verbund parallel zusammengeschaltet sind, derart, dass die Gasbeaufschlagung des einen Reaktors erhöht werden kann, indem in diesen Reaktor zusätzlich auch die Gasmenge eines anderen Reaktors verwendet wird.

8. Verfahren nach einem der vorangehenden Ansprüche,
**dadurch gekennzeichnet, dass** die Temperatur des in den Reaktor einzubringenden Gases zur Heizung des Reaktorinhalts eingestellt wird.

9. Verfahren nach einem der vorangehenden Ansprüche,
**dadurch gekennzeichnet, dass** eine Kühlung zur Regelung der Temperatur des Gases auf das zulässige Maß vorgenommen wird.

10. Verfahren nach Anspruch 9,
**dadurch gekennzeichnet, dass** die bei der Kühlung gewonnene Gaswärme abgezogen wird und diese Energie dem Prozess an zulässiger Stelle wieder zugeführt wird.

11. Verfahren nach einem der vorangehenden Ansprüche,
**dadurch gekennzeichnet, dass** alle außerhalb des Reaktors befindlichen Bestandteile wärmeisoliert sind.

## Geänderte Patentansprüche

### Geänderte Patentansprüche gemäss Regel 86(2) EPÜ.

**1.** Verfahren zur feinvolumigen Vermischung von Substraten in anaeroben Reaktoren mit Flachböden durch flächenartige bodennahe Gaseinblasung über im Wesentlichen die gesamte horizontale Querschnittsfläche,
wobei die Gaseinblasung in, den Boden im Wesentlichen vollständig abdeckende Sektionen (S) aufgeteilt ist, die steuerbar im dynamischen Wechsel mit Gas beaufschlagt werden, derart, dass an jedem beaufschlagten Volumenpunkt des Reaktors eine lokale Mikroturbulenz hervorgerufen wird.

**2.** Verfahren nach Anspruch 1,
**dadurch gekennzeichnet, dass** die Ansteuerung der Sektionen (S) zur Gasbeaufschlagung durch eine Bestimmung der Gasmenge durch die einzelnen Sektionen erfolgt.

**3.** Verfahren nach Anspruch 1 oder 2,
**dadurch gekennzeichnet, dass** die Ansteuerung der Sektionen (S) zur Gasbeaufschlagung im zeitlichen Wechsel erfolgt.

**4.** Verfahren nach Anspruch 3,
**dadurch gekennzeichnet, dass** der zeitliche Ansteuerungswechsel durch einen/mehrere frequenzgeregelte(n) Gasverdichter erfolgt.

**5.** Verfahren nach einem der vorangehenden Ansprüche,
**dadurch gekennzeichnet, dass** der Substrateintrag in den Reaktor im Wesentlichen gleichmäßig oben über die gesamte horizontale Reaktor-Querschnittsfläche erfolgt, um eine vertikal nach unten gerichtete Pfropfenströmung des Substrats zu ermöglichen.

**6.** Verfahren nach einem der vorangehenden Ansprüche,
**dadurch gekennzeichnet, dass** die in das Substrat einzubringende Gasmenge bei zunehmender Viskosität des Substrats erhöht ist.

**7.** Verfahren nach einem der vorangehenden Ansprüche,
**dadurch gekennzeichnet, dass** mehrere Reaktoren in einem Verbund parallel zusammengeschaltet sind, derart, dass die Gasbeaufschlagung des einen Reaktors erhöht werden kann, indem in diesen Reaktor zusätzlich auch die Gasmenge eines anderen Reaktors verwendet wird.

**8.** Verfahren nach einem der vorangehenden Ansprüche,
**dadurch gekennzeichnet, dass** die Temperatur des in den Reaktor einzubringenden Gases zur Heizung des Reaktorinhalts eingestellt wird.

**9.** Verfahren nach einem der vorangehenden Ansprüche,
**dadurch gekennzeichnet, dass** eine Kühlung zur Regelung der Temperatur des Gases auf das zulässige Maß vorgenommen wird.

**10.** Verfahren nach Anspruch 9,
**dadurch gekennzeichnet, dass** die bei der Kühlung gewonnene Gaswärme abgezogen wird und diese Energie dem Prozess an zulässiger Stelle wieder zugeführt wird.

**11.** Verfahren nach einem der vorangehenden Ansprüche,
**dadurch gekennzeichnet, dass** alle außerhalb des Reaktors befindlichen Bestandteile wärmeisoliert sind.
